# EUROPÄISCHE PATENTANMELDUNG

(11) **EP 1 977 752 A1**
(43) Veröffentlichungstag der Anmeldung: **08.10.2008**
(21) Anmeldenummer: 07004202.3
(22) Anmeldetag: 01.03.2007
(51) Int. Cl.: A61K 31/57, A61K 31/573, A61P 15/00

(54) **Pharmazeutische Zubereitung zur Verminderung der Endometriose**

(71) Anmelder: Bayer Schering Pharma Aktiengesellschaft, 13353 Berlin (DE)
(72) Erfinder: Seitz, Christian, 15738 Zeuthen (DE); Wasserfall, Annemarie, 13505 bERLIN (DE); Zimmermann, Holger, 14612 Falkensee (DE)
(74) Vertreter: Cramer, Eva-Maria

(57) **Zusammenfassung**

Eine pharmazeutische Zusammensetzung zur Behandlung der Endometriose enthält ein Gestagen mit antiandrogener Wirksamkeit, vorzugsweise 17α-Cyanomethyl-17-β-hydroxyestra-4,9-dien-3on (Dienogest), Cyproteronacetat oder Chlormadinonacetat in einer täglichen Dosis, die maximal das zweifache der Ovulationshemmdosis beträgt, gemeinsam mit einem oder mehreren pharmazeutisch annehmbaren Hilfsstoffen/Trägern.
Die erfindungsgemäße pharmazeutische Zusammensetzung realisiert sowohl die Verminderung der Endometriose als auch das Fehlen einer negativen Beeinflussung des Knochenstoffwechsels und der Knochendichte und vermag gleichzeitig die von den konventionellen Arzneimitteln bekannten Nebenwirkungen (z. B. Hitzewallungen, Akne, negative Beeinflussung des Lipidprofils) im erträglichen Maße zu halten. Die pharmazeutische Zusammensetzung ist daher für die Langzeitapplikation geeignet.

## Beschreibung

### Technisches Gebiet

Die Erfindung betrifft eine pharmazeutische Zusammensetzung zur Verminderung der Endometriose, welche ein Gestagen mit antiandrogener Wirksamkeit in einer täglichen Dosis, die maximal das zweifache der Ovulatiorishemmdosis beträgt, gemeinsam mit einem oder mehreren pharmazeutisch annehmbaren Hilfsstoffen/Trägern enthält. Die Erfindung realisiert auch ein monophasisches Präparat, welches keine negative Beeinflussung des Knöchenstoffwechsels bewirkt. Dieses Präparat ist daher für eine Langzeitanwendung geeignet. Gleichwohl ermöglicht die Verwendung von Gestagenen mit antiandrogener Wirksamkeit in obiger Dosis zur Herstellung eines pharmazeutischen Präparates zur Prophylaxe und/oder Therapie der Endometriose die aus konventionellen pharmazeutischen Zusammensetzungen bekannten Nebenwirkungen, wie beispielsweise Hitzewallungen, Akne, Änderung des Lipidprofils im erträglichen Maße zu halten.

### Stand der Technik

Die Endometriose ist eine chronische, gynäkologische Erkrankung, die primär bei 5-20% der Frauen im gebärfähigen Alter vorkommt. In der Fachliteratur wird die Endometriose definiert als das Vorkommen von Endometrium oder endometriumähnlichem Gewebe außerhalb des Cavum uteri. Typische Beschwerdebilder der Endometrioseerkrankung sind die Dysmenorrhoe. Dyspareunie und Schmerzen beim Stuhlgang. Endometriose-Patientinnen klagen häufig über Schmerzen im Beckenbereich. Unterleibsschmerzen, die in der 2. Zyklushälfte auftreten, gefolgt von einer schmerzhaften Regelblutung und anschließender Beschwerdefreiheit bis Mitte des folgenden Zyklus lassen häufig an eine Endometrioseerkrankung denken, aber auch dauerhafte Schmerzen sind nicht selten. Allerdings haben ca 30- 40 % der Endometriose Erkrankten keine Beschwerden. Die Erkrankung wird dann nur zufällig im Zusammenhang mit anderen diagnostischen Maßnahmen festgestellt. In ca. 50-60% wird die Diagnose "Erkrankung an Endometriose" als zufällige Diagnose bei der Abklärung einer Sterilität gestellt.

Aus der Fach- und Patentliteratur ist bekannt, Endometriose medikamentös mit Danazol, einem Derivat von 17α-Ethinyltestosteron, GnRH-Agonisten, Ge stagen/Estrogen-Kombinationen oder Gestagen-Monopräparaten zu behandeln.

US 6,569,845 offenbart die Behandlung von angiogenen Erkrankungen mit Dienogest in einer täglichen Dosis von 0,5 bis 10 mg. Entsprechende, als Beispiele ausgewiesene pharmazeutische Zusammensetzungen, die man weitläufig auch zur Behandlung von Endometriose einsetzen könnte, besitzen einen Dienogest-Gehalt von 400 mg bis 2 g.

Moore, C. *et al.,* The treatment of endometriosis, Drugs of Today 1999, 35 (Suppl C): 41-52 untersuchten in klinischen Studien die Wirksamkeit von Dienogest bei der Behandlung von Endometriose vergleichsweise zum Behandlungsregime mit Danazol oder GnRH-Agonisten. Den Endometriose Betroffenen wurden 24 Wochen 2 mg Dienogest pro Tag verabreicht. Das Ergebnis der Behandlung ist vergleichbar mit dem Ergebnis einer Behandlung mit Danazol oder GnRH-Agonisten- Bis zu 90 % der Betroffenen berichteten über irreguläre Blutungen, jedoch keine berichtete über unerträgliche Blutungen. Die Wirksamkeit der Standardbehandlung mit Danazol wird durch signifikante androgene Effekte geschmälert, während GnRH-Agonisten mit "menopausalen Symptomen" in Zusammenhang stehen.

Schweppe, K.-W, Stellenwert der Gestagene, Zentralbl Gynakol 2003, 125: 276-280 erklärt, dass bei kontinuierlicher oraler Gestagenbehandlung (beispielsweise mit Medroxyprogesteronacetat, Dienogest, Dydrogesteron, Lynestrenol in einer täglichen Dosierung von 5 mg bis 20 mg, als niedrig dosiert bezeichnet und eingestuft als wirksames Behandlungsprinzip bei Endometriose bedingten Symptomen) niedrige Estrogenspiegel zu verzeichnen sind. Es resultieren häufig Schmier- und Zwischenblutungen. Dies zwingt zur Dosiserhöhung und/oder Estrogenzugabe. Rezidivraten liegen langfristig über 50 %.

Eine Sicherheitsinformation von 2005 zu einem Medroxyprogesteronacetat-Produkt zeigt auf, dass Gestagen-Monopräparate besonders bei Langzeitbe-handlung negative Wirkung auf die Knochendichte ausüben können

In Kombination mit Estrogenen dagegen üben einige Gestagene einen positiven Einfluss auf den Knochenstoffwechsel aus.

Eine weitere Sicherheitsinformation, NDA 21-584, FDA 22.03.2005, zu deposubQ provera 104^{™} (Medroxyprogesteronacetat i.m. - 104 mg/0.65 ml), verweist darauf, dass Frauen, welche dieses Präparat verwenden, einen Kno chenmineraldichteverlust erleiden, der sich mit der Dauer der Verwendung des Präparates vergrößert und nicht mehr komplett reversibel ist.

Knauthe, R und Habenicht U.F., Levonorgestrel has benefical effects, Exp Clin Endocrinol diabetes 106 (1998) Suppl 1: 37 zeigen bereits 1998 auf, dass dabei die partielle androgene Wirkung eines Gestagens (Levonorgestrel) und nicht die gestagene Aktivität ausschlaggebend ist für diesen positiven Einfluss auf den Knochenstoffwechsel. Auch Kuhl, H., Klimakterium, Postmenopause und Hormonsubstitution, 3. Aufl., Bremen. UNI-MED, 2006, 117 betont, dass bestimmte Gestagene über ihre androgene Partialwirkung wirksam werden. Ferner erklärt Kuhl, dass Androgene die positive Wirkung der Estrogene auf die Knochendichte erheblich verstärken.

### Darstellung der Erfindung

Aufgabe der Erfindung ist eine pharmazeutische Zusammensetzung mit möglichst niedrigem steroidalen Gehalt zur Verminderung der Endometriose, wobei die Zusammensetzung gleichzeitig keine negativen Einfluss auf die Knochendichtelden Knochenstoffwechsel ausüben sollte.

Es wurde nun gefunden, dass die Minderung der Endometriose durch eine pharmazeutische Zusammensetzung mit niedriger hormoneller Dosierung, nämlich ein Gestagen mit antiandrogener Wirksamkeit, dessen tägliche Dosis maximal das zweifache der Ovulationshemmdosis beträgt, und einen oder mehrere pharmazeutisch annehmbare Hilfsstoffe/Träger erreicht werden kann.

Gleichzeitig realisiert die pharmazeutische Zusammensetzung bzw. deren Verwendung oder das entsprechende monophasische Präparat, dass neben der Minderung der Endometriose überraschenderweise keine negative Beeinflussung des Knochenstoffwechsel, so dass keine Abnahme/Verringerung der Knochendichte zu verzeichnen ist.

Gleichwohl gelingt es der pharmazeutischen Zusammensetzung bzw deren Verwendung oder das entsprechende monophasische Präparat ütierraschenderweise, die von den konventionellen Arzneimitteln zur Behandlung der Endometriose bekannten Nebenwirkungen, z.B. Hitzewallungen, Änderung des Lipidprofils im erträglichen Maße zu halten.

Erfindungsgemäß sind die verwendeten Gestagene mit antiandrogener Wirksamkeit Dienogest, Cyproteronacetat oder Chlormadinonacetat.

Die tägliche Dosis an Dienogest als maximal das zweifache der Övulationshemmdosis beträgt maximal 2 mg. Auch Cyproteronacetat oder Chlormadinonacetat werden erfindungsgemäß in einer täglichen Dosierung von maximal dem zweifachen der Ovulationshemmdosis eingesetzt. Die Ovulatäonshemmdosis von Cyproteronacetat beträgt 1 mg, die von von Chlormadinonacetat 1.7 mg.

Die tägliche Dosis an den Gestagenen kann 1 mal das maximal zweifache der Ovulationshemmdosis oder 2 mal gleiche Dosis an maximal der Hälfte der Ovulationshemmdosis betragen.

Auch wird die Aufgabe erfindungsgemäß durch eine pharmazeutische Zusammensetzung gelöst; enthaltend getrennt verpackte und einzeln entnehmbare Tagesdosiseinheiten, welche für die Dauer von 28 oder 30 aufeinander folgenden Tagen in einer Verpackungseinheit eingebracht sind , wobei die Tagesdosiseinheiten maximal 2 mg Dienogest oder eine äquivalente Menge an Cyproteronacetat oder Chlormadinonacetat gemeinsam mit einem oder mehreren pharmazeutisch annehmbaren Hilfsstoffen/Trägern enthalten. Vorzugsweise sind die Verpackungseinheiten 2 Blister zu je 14 oder 15 Tagesdosiseinheiten.

Es wurde gefunden, dass die pharmazeutische Zusammensetzung, welche zur Prophylaxe und/oder Therapie der Endometriose geeignet ist, überraschenderweise keinen negativen Einfluss auf den Knochenstoffwechsel und die Knochendichte sowie das Lipidprofil ausübt. Die pharmazeutische Zusammenstzung ist daher überraschenderweise für die Langzeitapplikation geeignet ist, besonders mit einer kontinuierlichen Verabreichung der Dosierungsform für die Dauer von mindestens 169 Tagen oder 25 Wochen bis mehreren Jahren, vorzugsweise mehr als 2 Jahren.

Erfindungsgemäß wird die Aufgabe auch durch ein Kit gelöst, welches mindestens 28, bevorzugt 30 tägliche Dosiseinheiten von der maximalen zweifachen Ovulationshemmdosis eines Gestagens mit partieller antiandrogener Wirkung, bevozugt Dienogest, Cyproteronacetat oder Chlormadinonacetat gemeinsam mit einem oder mehreren pharmazeutisch annehmbaren Hilfsstoffen/Trägern enthält.

Des weiteren betrifft die vorliegende Erfindung die Verwendung von Gestagenen mit antiandrogener Wirksamkeit in einer täglichen Dosis, die maximal das zweifache der Ovulationshemmdosis beträgt, zur Herstellung eines pharmazeutischen Präparates zur Prophylaxe und/oder Therapie der Endometriose. wobei gefunden wurde, dass überraschenderweise keine negative Beeinflussung des Knochenstoffwechsels, so dass keine Abnahme der Knochendichte zu verzeichnen ist. Gleichzeitig werden die von den konventionellen Arzneimitteln zur Behandlung der Endometriose bekannten Nebenwirkungen, z.B. Hitzewallungen, Änderung des Lipidprofils im erträglichen Maße gehalten. Weitere erfindungsgemäße Ausführungsformen ergeben sich aus den Merkmalen der Unteransprüche 9,10. 11 oder 12.

Das pharmazeutische Präparat kann in Form von Tabletten, Kapseln, Dragees, Wafer, Transdermalen-Therapie-Systemen, Ampullen, Suppositorien, Gelen, Salben, Implantaten, Vaginalringen oder Nasenspray vorliegen.

Dabei ist die von den nichtoralen Formen des pharmazeutischen Präparates, wie Transdermales-Therapie-System, Ampulle, Suppositorie, Gel, Salbe, Implantat, Vaginalring oder Nasenspray abgegebene tägliche Gestagendosis äquivalent zur maximal das zweifache der Ovulationshemmdosis betragende täglichen Dosiseinheit bei den oralen Formen.

Weiterhin betrifft die Erfindung ein monohasisches Präparat zur Minderung der Endometriose, welches mindestens 28 Dosierungseinheiten bevorzugt 30 Dosierungseinheiten, gegebenenfalls in je 2 Blistern zu 14 oder 15 Dosierungseinheiten, jeweils enthaltend ein Gestagen mit antiandrogener Wirsamkeit mit der maximal zweifachen Ovulationshemmdosis, ausgewählt aus Dienogest, Cyproteronacetat oder Chlormadinonacetat umfasst.

Auch betrifft die Erfindung ein monohasisches Präparat zur Minderung der Endometriose, welches obige Dosierungseinheiten , jeweils enthaltend ein Gestagen mit antiandrogener Wirksamkeit mit der maximal zweifachen Ovulationshemmdosis, ausgewählt aus Dienogest, Cyproteronacetat oder Chlormadinonacetat, wobei die Dosierungseinheiten kontinuierlich mindestens 169 bis mehr als 730 Tagen verabreicht werden, umfasst.

Das monophasische Präparat ist zur Prophylaxe und/oder Therapie der Endometriose geeignet, bzw. vermindert die Endometriose; beeinflusst nicht negativ den Knochenstoffwechsel/ die Knochendichte und hält sowohl die bekannten Endometriosetherapiebedingten Nebenwirkungen (verminderte Knochendichte, Hitzewallungen, verändertes Lipidprofil) im erträglichen Maße. Es ist deshalb zur Langzeittherapie geeignet.

### Ausführungsbeispiele

### Beispiel 1

Es werden Tabletten mit folgender Zusammensetzung hergestellt:

| | |
|---|---|
| Dienogest, micronisiert min, 99% ≤ 20 µm, 100 % <30 µm | 2.000 mg |
| Lactose-Monohydrat | 62.800 mg |
| Microkristalline Cellulose | 18.000 mg |
| Kartoffelstärke | 36.000 mg |
| Povidon K 25 | 8.100 mg |
| Magnesiumstearat | 1.350 mg |
| Talkum | 4.050 mg |
| Crospovidon | 2.700 mg |

Dienogest wird micronisiert mit einer mittleren Teilchengröße von 20 µm eingesetzt und mit Lactose-Monohydrat, Microkristalliner Cellulose und Kartoffelstärke vermischt. Das Povidon K 25 wird während der Granulierung eingesprüht. Nach Trocknen und Zumischen von Talkum, Crospovidon und Magnesiumstearat wird die Mischung aus den Substanzen zu Tabletten; mit einem Durchmesser von 7 mm und einer Masse von 135 mg gepresst.

### Beispiel 2

In einer klinischen Studie wurden 252 Frauen mit laparoskopisch diagnostizierter Endometriose über einen Zeitraum von 6 Monaten entweder mit dem GnRH-Agonisten Leuprorelin Acetat (LA) 3,75 mg s.c. alle 4 Wochen oder mit 2mg/d oral des Gestagens Dienogest (DNG) behandelt. 128 Patientinnen wurden in die LH-Gruppe und 124 Patientinnen in die DNG-Gruppe randomisiert. Die Wirksamkeit der jeweiligen Therapie wurde u.a. mittels einer von der Patientin auszufüllenden Schmerzskale (Visual Analogue Scale, VAS) untersucht. Am Ende der Behandlung zeigte sich in beiden Vergleichsgruppen eine ähnliche Reduktion der Schmerzen im Vergleich zum Studienbeginn (-47,5 mm für DNG; -46,0 mm für LA). Die statistische Analyse zeigte die Nicht-Unterlegenheit von DNG gegenüber LA.

Weiterhin wurden häufige Nebenwirkungen hormoneller Therapieverfahren bei Endometriose erfasst:
In beiden Behandlungsgruppen traten Veränderungen der Menstruationsblutung - häufig als ausbleibende Regelblutung oder in Form leichter Zwischenblutungen - auf, die aber nur bei wenigen Patientinnen zum Abbruch der Therapie führten.
Hitzewallungen, ein typisches Symptom bei Östrogenmangel, traten in der DNG-Gruppe wesentlich seltener auf (0,89 Tage mit Hitzewallungen / Woche) als in der LH-Gruppe (4,23 Tage / Woche). Zusätzlich verringerte sich die Symptomatik in der DNG-Gruppe im Verlauf der 6-monatigen Therapie, während sie unter LA zunahm.

In einer Untergruppe von Patientinnen wurde der Einfluss beider Therapien auf den Knochenstoffwechsel untersucht. Am Ende der 6-monatigen Therapie zeigte sich ein statistisch signifikanter Unterschied zum Vorteil von DNG: Unter DNG war die Knochendichte im Vergleich zum Studienbeginn nahezu unverändert (+0,25%), während in der Vergleichsgruppe ein deutlicher Abfall (-4,0 %) aufgetreten war. Diese Ergebnisse wurden durch Laborparameter zur Bestimmung des Knochenstoffwechsels bestätigt, die auf eine erhöhte Knochenresorption unter Therapie mit LA hinweisen.

Die Östrogenwerte blieben unter Therapie mit DNG weitgehend unverändert (Mittelwert vor Therapie: 256,3 pmol/L; Studienende: 249,9 pmol/L), während unter LA ein deutlicher Abfall auftrat (vor Therapie: 299,0 pmol/L; Studienende: 68,5 pmol/L). Die weiteren untersuchten Laborwerte zur Sicherheit ergaben in keiner der beiden Behandlungsgruppen signifikante Veränderungen.

Insgesamt waren beide Behandlungen in Bezug auf die Wirksamkeit gleichwertig, während sich in Bezug auf Östrogenmangel-bedingte Nebenwirkungen wie Hitzewallungen und verringerte Knochendichte deutliche Vorteile für DNG abzeichnen.

## Patentansprüche

**1.** Pharmazeutische Zusammensetzung zur Verminderung der Endometriose, enthaltend ein Gestagen mit antiandrogener Wirksamkeit in einer täglichen Dosis, die maximal das zweifache der Ovulationshemmdosis beträgt, gemeinsam mit einem oder mehreren pharmazeutisch annehmbaren Hilfsstoffen/Trägern.

**2.** Pharmazeutische Zusammensetzung nach Anspruch 1, **dadurch gekennzeichnet, dass** die Gestagenkomponente 17α-Cyanomethyl-17-ß-hydroxyestra-4,9-dien-3on (Dienogest), Cyproteronacetat oder Chldrmadinonacetat ist.

**3.** Pharmazeutische Zusammensetzung nach Anspruch 2, **dadurch gekennzeichnet, dass** die tägliche Dosis 2 mg Dienogest oder eine äquivalente Menge an Cyproteronacetat oder Chlormadinonacetat beträgt.

**4.** Pharmazeutische Zusammensetzung nach Anspruch 3, enthaltend getrennt verpackte und einzeln entnehmbare Tagesdosiseinheiten, weiche für die Dauer von mindestens 28 , vorzugsweise 30 aufeinander folgenden Tagen in einer Verpackungseinheit eingebracht sind, wobei die Tagesdosiseinheiten 2 mg Dienogest oder eine äquivalente Menge an Cyproteronacetat oder Chlormadinonacetat gemeinsam mit einem oder mehreren pharmazeutisch annehmbaren Hilfsstoffen/Trägern enthalten.

**5.** Pharmazeutische Zusammensetzung nach Anspruch 4 mit einer kontinuierlichen Verabreichung der Dosierungsform für die Dauer von mindestens 1ö9 Tagen oder 25 Wochen bis mehreren Jahren, vorzugsweise mehr als 2 Jahren, zur Prophylaxe und/oder Therapie der Endometriose.

**6.** Pharmazeutische Zusammensetzung nach den Ansprüchen 1,2,3,4 oder 5 ohne negative Beeinflussung des Knochenstoffwechsels und damit Verringerung der Knochendichte.

**7.** Kit, enthaltend mindestens 28 tägliche Dosiseinheiten einer pharmazeutischen Zusammensetzung nach Anspruch 1,2 oder 3.

**7.** Kit nach Anspruch 6, enthaltend 30 tägliche Dosiseinheiten einer pharmazeutischen Zusammensetzung.

**8.** Verwendung von Gestagenen mit antiandrogener Wirksamkeit in einer täglichen Dosis, die maximal das zweifache der Ovulationshemmdosis beträgt, zur Herstellung eines pharmazeutischen Präparates zur Prophylaxe und/oder Therapie der Endometriose.

**9.** Verwendung von Gestagenen nach Anspruch 8, **dadurch gekennzeichnet, dass** die Gestagenkomponente 17α-Cyanomethyl-17-ß-hydroxyestra-4,9-dien-3on (Dienogest), Cyproteronacetat oder Chlormadinonacetat ist.

**10.** Verwendung von Gestagenen nach Anspruch 8 oder 9, **dadurch gekennzeichnet, dass** die tägliche Dosis 2 mg Dienogest oder eine äquivalente Menge an Cyproteronacetat oder Chlormadinonacetat beträgt.

**11.** Verwendung von Gestagenen nach Anspruch 10, zur Herstellung eines pharmazeutischen Präparates mit kontinuierlicher Verabreichung für die Dauer von mindestens 169 Tagen oder 25 Wochen bis mehreren Jahren, vorzugsweise mehr als 2 Jahren, zur Prophylaxe und/oder Therapie der Endometriose.

**12.** Verwendung von Gestagenen nach den Ansprüchen 8, 9, 10 oder 11 ohne negative Beeinflussung des Knochenstoffwechsels und damit Verringerung der Knochendichte.

**13.** Verwendung von Gestagenen nach Anspruch 8, 9, 10, 11 oder 12, **dadurch gekennzeichnet, dass** das pharmazeutische Präparat in Form von Tabletten, Kapseln, Dragees, Wafer, Transdermaien-Therapie-Systemen, Ampullen, Suppositorien, Gelen, Salben, implantaten, Vaginalringen oder Nasenspray vorliegt.
